(19) Europäisches Patentamt
European Patent Office
Office européen des brevets

(11) Veröffentlichungsnummer: **0 032 657**
**B1**

(12) # EUROPÄISCHE PATENTSCHRIFT

(45) Veröffentlichungstag der Patentschrift: **25.05.83**

(51) Int. Cl.³: **C 07 C 13/61,** C 07 C 2/04, C 09 D 3/64

(21) Anmeldenummer: **81100019.9**

(22) Anmeldetag: **05.01.81**

(54) **Neuer Kohlenwasserstoff, Verfahren zu seiner Herstellung und seine Verwendung als Reaktivverdünner für luft-trocknende Lacke.**

(30) Priorität. **18.01.80 DE 3001739**

(43) Veröffentlichungstag der Anmeldung: **29.07.81 Patentblatt 81/30**

(45) Bekanntmachung des Hinweises auf die Patenterteilung: **25.05.83 Patentblatt 83/21**

(84) Benannte Vertragsstaaten: **AT DE FR GB IT NL SE**

(56) Entgegenhaltungen: **US-A-3 183 249**

(73) Patentinhaber: **BAYER AG, Zentralbereich Patente, Marken und Lizenzen, D-5090 Leverkusen 1, Bayerwerk (DE)**

(72) Erfinder: **Eimers, Erich, Dr., Kreuzbergstrasse 136, D-4150 Krefeld (DE)**
Erfinder: **Dhein, Rolf, Dr., Deswatinesstrasse 30, D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 032 657 B1

BUNDESDRUCKEREI BERLIN

Neuer Kohlenwasserstoff, Verfahren zu seiner Herstellung und seine Verwendung als Reaktivverdünner für lufttrocknende Lacke

Die Erfindung betrifft ein Addukt von Butadien-1.3 und Dicyclopentadien, ein Verfahren zur Herstellung des Addukts durch homogene Katalyse und seine Verwendung als Reaktivverdünner für lufttrocknende Lacke.

Reaktivverdünner sind niedrigviskose Stoffe, die harzartige Bindemittel verdünnen und dadurch dem Lack die zu seiner Applikation notwendige Viskosität verleihen, zur Mischpolymerisation oder Mischkondensation mit dem Lackharz befähigte funktionelle Gruppen enthalten und beim Härtungsvorgang zum überwiegenden Teil — jeweils in Abhängigkeit von ihrer Flüchtigkeit — Bestandteil des gehärteten Lackfilms werden.

Reaktivverdünner für lufttrocknende Lacke müssen also funktionelle Gruppen, z. B. aktivierte, vorzugsweise konjugierte, Doppelbindungen tragen, die an dem durch den Sauerstoff der Luft induzierten Vernetzungsvorgang teilnehmen können. Alloocimen, ein derartiger Reaktivverdünner, ist bereits seit einiger Zeit auf dem Markt, wegen seines auch in vielfacher Verdünnung intensiven unangenehmen Geruchs und seiner relativ hohen Flüchtigkeit nur begrenzt anwendbar.

Addukte von Butadien-1,3 an Dicyclopentadien (DCP) sind bekannt. So weiß man, daß sich in Gegenwart von metallorganischen Komplexverbindungen des nullwertigen Nickels bei einem hohen Butadienüberschuß 2 Moleküle Butadien an ein Molekül DCP anlagern und durch Ringschluß der beiden Butadienylreste ein neuer annelierter Ring entsteht. Die entstehenden Mischoligomeren enthalten ausschließlich isolierte Doppelbindungen und lagern sich schon bei relativ niedrigen Temperaturen im Sinne einer Cope-Umlagerung um; außerdem sind die Verbindungen bei Raumtemperatur kristallin; vgl. Rolf-Volker Meyer, »Mischoligomerisation von Butadien mit gespannten Olefinen an Nickelkatalysatoren«, Diss. Bochum, 1973.

Butadien läßt sich mit anderen Verbindungen unter Erhaltung seines Systems konjugierter Doppelbindungen mittels homogener Katalyse umsetzen allerdings nur mit solchen Verbindungen, die besonders aktivierte Doppelbindungen besitzen. So erhält man durch Erhitzen äquimolarer Mengen von Butadien-1.3 und Norbornadien, das dank extremer Ringspannung ein stark aktiviertes Olefin darstellt, in Gegenwart niederwertiger Kobaltkomplexe im Autoklaven ein 1 : 1-Addukt mit einem Paar konjugierter Doppelbindungen; das Hauptisomere fällt in einer Ausbeute von ca. 50% an (A. Takahashi et al., Chem. Comm. 1970, No. 22, 1473). Eigene Versuche haben ergeben, daß wegen der hohen Flüchtigkeit dieses Addukts bei Verwendung als Reaktivverdünner ein vergleichsweise geringer Anteil im gehärteten Lackfilm verbleibt.

Es wurde nun gefunden, daß sich auch DCP mit Butadien-1.3 zu einem 1 : 1-Addukt mit konjugierten Doppelbindungen umsetzen läßt, überraschenderweise in hoher Ausbeute, was aufgrund der niedrigen Reaktivität von DCP nicht vorhersehbar war. Die Umsetzung gelingt in befriedigender Weise nur unter Bedingungen, die von den zur Herstellung der oben erwähnten Addukte wesentlich abweichen.

Das Prinzip des Herstellungsverfahrens besteht im Gegensatz zu den oben erwähnten Verfahren darin, während der gesamten Umsetzungsdauer die Konzentration des Butadiens möglichst niedrig zu halten. Dies läßt sich dadurch erreichen, daß man zumindest den größten Teil des gasförmigen Butadiens-1,3 während eines längeren Zeitraumes und höchstens so schnell, wie es verbraucht wird, in eine Lösung eines Katalysators in erwärmtem DCP einleitet. Die Ausbeute beläuft sich auf bis zu 90%, bezogen auf umgesetztes DCP. Durch Destillation läßt sich das erhaltene Codimere leicht aus der Reaktionsmischung abtrennen.

Das massenspektrometrisch bestimmte Molekulargewicht des Codimeren beträgt 186; dies entspricht dem erwarteten Wert für ein 1 : 1-Addukt. Das IR-Spektrum zeigt eine isolierte und zwei konjugierte Doppelbindungen. Mit Maleinsäureanhydrid bildet sich in exothermer Reaktion ein Diels-Alder-Addukt. Aufgrund dieser Daten werden dem erhaltenen Codimeren die Strukturen

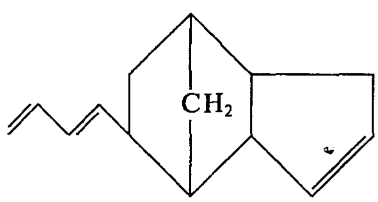

(I)

und/oder

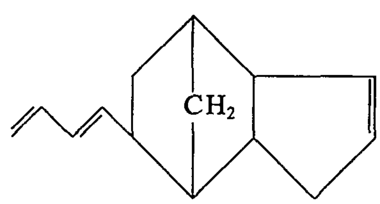

(II)

zugeordnet.

Gegenstand der Erfindung sind also 1 : 1-Codimere eines Molekulargewichts von 186 aus DCP und Butadien-1,3 der Struktur I und/oder II.

Weiterer Gegenstand der Erfindung ist ein Verfahren zur Herstellung von 1 : 1-Codimeren aus DCP und Butadien-1,3 der Struktur I und/oder II, dadurch gekennzeichnet, daß man gasförmiges Butadien-1,3 mit DCP in Gegenwart einer kobaltorganischen Komplexverbindung bei 40—100,

vorzugsweise 50—70, °C reagieren läßt, wobei man den überwiegenden Anteil Butadien-1,3 höchstens im Maße seiner Reaktionsgeschwindigkeit zufügt.

Nach einer bevorzugten Verfahrensweise wird das Butadien-1,3 in einer Menge von 0,05—0,5 Mol/h pro Mol vorgelegtes DCP zudosiert. Das Molverhältnis Butadien/DCP beträgt vorzugsweise 0,1 bis 2, insbesondere 1/3 bis 1. Der Druck beträgt vorzugsweise 1 bis 1,5 bar.

Weiterer Gegenstand der Erfindung ist die Verwendung der erfindungsgemäßen Codimeren als Reaktivverdünner für lufttrocknende Lacke.

Bevorzugte Katalysatoren für das erfindungsgemäße Verfahren sind die organischen Komplexverbindungen des null- und einwertigen Kobalts, wie z. B. Cyclooctenylcyclooctadienyl-Kobalt, 3-Methylheptadienyl-Kobalt, Bis-(cyclooctadien)-ethyl-kobalt, Trisallylkobalt, Tetrakistrisphenylphosphinkobalt, Kobaltbis-1,2-(diphenylphosphinethan)-hydrid, Kobaltbis-1,2-diphenylphosphinmethan und ähnliche — also Komplexverbindungen, wie sie z. B. in der DE-AS 1 191 375 oder in Internat. Congress Pure Appl. Chem. 6, 265 ff (1971) beschrieben sind. Die Katalysatoren werden in der Regel in einer Menge von 1—15 mMol/Mol Butadien eingesetzt.

Die Katalysatoren können auch in bekannter Weise in situ hergestellt werden, indem man in vorgelegtem DCP in der Reaktionsmischung Salze des Kobalts, wie z. B. Kobaltacetat, -chlorid oder -acetylacetonat mit starken Reduktionsmitteln, wie z. B. Natriumborhydrid, Natriumamalgam, Kalium, Natrium, Aluminiumdiethylchlorid, Oxialkylaluminiumdialkyl, Aluminiumtriethyl usw. in Gegenwart von komplexbildenden Liganden, z. B. Butadien, Allylverbindungen, Cyclooctadien, Cyclopentadien, Triphenylphosphin, Triphenylstibin, Pyridin umsetzt.

Das erfindungsgemäße Verfahren kann in An- oder Abwesenheit inerter Lösungsmittel durchgeführt werden. Bevorzugte Lösungsmittel sind aromatische Kohlenwasserstoffe wie Benzol, Toluol, cycloaliphatische Kohlenwasserstoffe wie Cyclohexan, aliphatische und cycloaliphatische Ether wie Dimethyl-, Diethylether, Tetrahydrofuran, Dioxan, Diethylenglykol, Glykol-1,2-dimethylether, Ester von Carbonsäuren mit 2—4 C-Atomen und Alkoholen mit 1—4 C-Atomen wie Ethyl-, Butylacetat und Glykolbisacetat.

Das erfindungsgemäße Verfahren soll unter Ausschluß von Sauerstoff und Feuchtigkeit durchgeführt werden; vgl. z. B. Methoden der organischen Chemie (Houben-Weyl), Bd. I/2, 4. Aufl., 326—390, Georg Thieme Verlag Stuttgart 1959.

Für die Verwendung als Reaktivverdünner kann das erfindungsgemäße Codimere in reiner oder angereicherter Form eingesetzt werden; so ist es z. B. möglich, solche Fraktionen des Reaktionsgemisches zu verwenden, in denen sich das Codimere in einer Konzentration von mindestens 60 Gew.-% befindet.

Als lufttrocknende Bindemittel, für die die erfindungsgemäßen Codimeren als Reaktivverdünner mit Vorteil eingesetzt werden können, dienen vorzugsweise Alkydharze, also fettsäure- bzw. ölmodifizierte Polyester, wie sie z. B. bei D. H. Solomon, The Chemistry of Organic Filmformers, S. 75—101, John Wiley & Sons Inc., New York 1967 beschrieben sind. Lufttrocknend sind die Alkydharze dann, wenn sie Reste mehrfach ungesättigter Fettsäuren, z. B. Linolsäure, Linolensäure, Eläostearinsäure, Ricinensäure und dgl. einkondensiert enthalten.

Der Reaktivverdünner kann auch in Kombination mit üblichen Lacklösungsmitteln, wie z. B. Benzin, Xylol, Naphtha, Diacetonalkohol usw. eingesetzt werden; 10—100 Gew.-% des Lacklösungsmittels sollen aus dem erfindungsgemäßen Codimeren bestehen.

Die lufttrocknenden Lacke können neben Bindemittel, Reaktivverdünner und gegebenenfalls inerten Lösungsmitteln natürlich auch anorganische und organische Pigmente, Hautverhinderungsmittel, wie z. B. Phenole oder Oxime, sowie Verlaufsmittel, wie z. B. Siloxanverbindungen enthalten. Zur Beschleunigung der Lufttrocknung können sie wirksame Schwermetallsalze, z. B. Octoate oder Naphthenate des Bleis, Mangans, Kobalts, Cers oder Zirkons, oder Mischungen derselben enthalten.

Der Festharzgehalt der lufttrocknenden Lacke kann in weiten Grenzen schwanken, beträgt aber vorzugsweise 20 bis 80 Gew.-%.

Die hohe Verträglichkeit des erfindungsgemäßen Codimeren mit lufttrocknenden Lacken zeigt sich in der niedrigeren Viskosität der Lösungen in Codimer, verglichen mit Lösungen in Lackbenzin. Das erfindungsgemäße Codimer eignet sich daher in hervorragender Weise zur Herstellung von Lacken mit hohem Harzgehalt. Beim Trocknungsvorgang wird das Codimer dann irreversibel im Lackfilm fixiert.

Das erfindungsgemäße Codimere eignet sich auch als Mischpolymerisationskomponente für Thermoplaste und Elastomere.

Die in den nachfolgenden Beispielen vorkommenden Prozentangaben bedeuten Gewichtsprozente; Teile bedeuten Gewichtsteile.

## Beispiele

### Beispiel 1

In einem luft- und feuchtigkeitsfreiem Medium (Schutzgas Argon) werden 3,4 g Kobalt-acetylacetonat in 270 g DCP gelöst. In dieser Lösung werden bei −20°C 13 g gasförmiges Butadien-1,3 unter leichtem Überdruck (ca. 15 Torr) kondensiert. Dann werden 3,8 g Triethylaluminium einpipettiert, die Mischung noch eine halbe Stunde bei dieser Temperatur belassen und anschließend wird die Reaktionsmischung auf 50°C aufgeheizt. Nach Erreichen dieser Temperatur werden im Verlaufe von 6 h 95 g Butadien-1,3 eingeleitet (0,14 Mol/h pro Mol DCP).

Nach Beendigung der Reaktion beträgt das Gewicht der Reaktionsmischung 310 g entsprechend einem Butadienumsatz von ca. 30%. Der Katalysator wird durch Zugabe von etwas Methanol zerstört. Anschließend wird das Reaktionsgemisch fraktioniert.

### Ergebnis der Fraktionierung

Fraktion 1:
174 g 36—60° C/0,1 Torr (nicht umgesetztes DCP)
Fraktion 2:
105 g 60—110° C/0,1 Torr
Destill.-Rückstand:
31 g (braunes, viskoses Öl)

Fraktion 2 wird nochmals destilliert:
Fraktion 3:
54,5 g 55—70° C/0,1 Torr
Fraktion 4:
39,2 g 70—80° C/0,1 Torr
Rückstand 11 g

Beide Fraktionen werden gaschromatographisch analysiert.

### Bedingungen

Säule:
4 m rostfreies Stahlrohr ∅1/8 mm
Füllung:
4% OV 101 auf Chromosorb G-AW-DMCS, Trägergas: Helium
Strömung:
25 ml/min., Detektorstrom: 150 mA

Temperaturen:
Injektor:
300° C, Detektor: 300° C, Ofen: 100—320° C, 8° C/min.,
Einspritzmenge:
0,002 ml
Registrierpapiervorschub:
0,5 cm/min.,

Ergebnis

| | DCP | Codimeres |
|---|---|---|
| Fraktion 3 | 87,6% | 1% |
| Fraktion 4 | 1% | 88,5% |

Die gaschromatographische Feinfraktionierung des Codimeren-Peaks zeigt eine Aufspaltung in 3 Komponenten, wobei es sich vermutlich um Stereoisomere handelt. Die 3 Komponenten zeigen im Massenspektrum alle die Molmasse 186. Bei der Aufnahme des IR-Spektrums einer destillativ auf 95% Reingehalt angereicherten Codimerenfraktion (vgl. Beispiel 2) ergeben sich folgende charakteristische Banden:

$-CH{=}CH-CH{=}CH_2$ $\begin{cases} 1645\ cm^{-1} \\ 1598 \end{cases}$

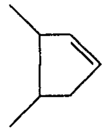 720 $cm^{-1}$

$-CH{=}CH_2$ $\begin{cases} 988\ cm^{-1} \\ 878 \end{cases}$

Ausbeute: 51%, bezogen auf umgesetztes DCP.

Beispiel 2

Analog Beispiel 1 werden 132 g DCP mit 3 g Cyclooctenylcyclooctadienylkobalt unter Argon auf 60° C erhitzt. Dann werden im Verlaufe von 6 h 54 g Butadien-1,3 eingeleitet (Einleitungsgeschwindigkeit 0,167 Mol/h pro Mol DCP). Nach beendeter Zugabe verbleiben 185 g eines braungefärbten Öls entsprechend einem Butadienumsatz von 92,6%. Bei der destillativen Aufbereitung des Rohrprodukts, die unter Zusatz von 0,1 g Phenothiazin erfolgt, werden 103 g nicht umgesetztes DCP zurückgewonnen. Nach Redestillation der Hauptfraktion werden bei einer Kopftemperatur von 70—78° C/0,1 Torr 27 g Addukt mit einem gaschromatographischen Reinheitsgrad von 95% erhalten. Ausbeute, bezogen auf umgesetztes DCP: 62,7%.

Beispiel 3

Analog Beispiel 1 werden in 462 g Dicyclopentadien, das 5,5 g Cyclooctenylcyclooctadienylkobalt enthält, in 6 h 108 g Butadien-1,3 eingeleitet (Einleitungsgeschwindigkeit 0,095 Mol/h pro Mol DCP). Die Temperatur während der Einleitung beträgt 65° C. Die erhaltene Menge an Rohprodukt beträgt 549 g.

Die destillative Aufarbeitung gemäß den vorhergehenden Beispielen ergibt 408 g nicht umgesetztes DCP sowie 77,2 g Codimerenfraktion mit einem Reingehalt von ca. 90%.

Ausbeute: 91,4%, bezogen auf umgesetztes DCP.

Beispiel 4

Verwendung des Codimeren als Reaktivverdünner

33 Teile der 70%igen Testbenzinlösung eines handelsüblichen Alkydharzes mit einem Gehalt von ca. 48% Sojaölfettsäuren (Alkydal® F 48, Bayer AG), werden mit 10 Teilen Codimeren (Reinheitsgrad 88%) gelöst. Zu dieser Lösung werden 2,23 Teile einer Sikkativlösung zugegeben.

Zusammensetzung der Sikkativlösung:

| | |
|---|---|
| 15% | Kobaltoctoatlösung (Metallgehalt 6%), |
| 62,7% | Bleioctoatlösung (Metallgehalt 24%), |
| 22,3% | Hautverhinderungsmittel. |

Die Viskosität der erhaltenen Lösung A beträgt 3260 mpas. Wird in dieser Lösung das Codimere durch die gleiche Gewichtsmenge Lackbenzin ersetzt, resultiert eine Lösung B mit einer Viskosität von 5540 mpas.

Filme der beiden Lösungen trocknen innerhalb einiger Stunden klebfrei auf. Der Verlust der Codimer enthaltenden Lacklösung A an flüchtigen Stoffen beträgt während der Trocknungszeit 27,4%. Der aus der Vergleichslösung B erhaltene Film gibt dagegen 47,5% seines Gewichts an flüchtigen Stoffen ab.

Daraus ergibt sich, daß ca. 80% des beanspruchten Reaktivverdünners bei der autoxidativen Trocknung im Film verbleiben.

Beispiel 5

Herstellung eines Weißlackes

182 Teile der 55%igen Lösung eines handelsüblichen Alkydharzes mit einem Gehalt von ca. 48% Sojaölfettsäuren (Alkydal® F 48, BAYER AG) in Benzin/Xylol (Gewichtsverhältnis 84 : 16) werden mit 81,6 Teilen Titandioxid-Pigment, 3,1 Teilen Calciumoctoat, 10 Teilen Aromatengemisch und 25 Teilen des erfindungsgemäßen Codimeren angerieben. In diese Masse werden 6,7 Teile der Sikkativlösung aus Beispiel 4, 3,8 Teile Siliconöl und 3 Teile Ethylglykolacetat eingerührt. Die erhaltene Lösung hatte eine Viskosität entsprechend einer Auslaufzeit von 81 sek. (DIN 53 211, DIN-Becher 4.) Der Festgehalt des Weißlackes beträgt 69,7%.

Der Weißlack trocknet in dünner Schicht innerhalb einiger Stunden zu einem klebfreien Lackfilm.

**Patentansprüche**

1. 1 : 1-Codimere eines Molekulargewichts von 186 aus Dicyclopentadien und Butadien-1,3 der Struktur

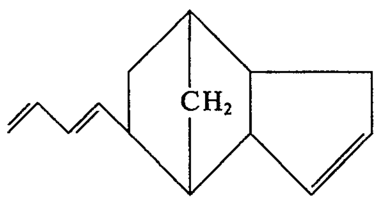

(I)

und/oder

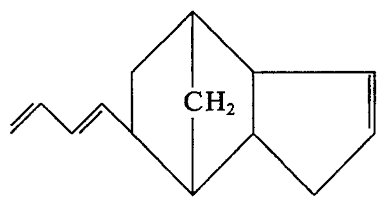

(II)

2. Verfahren zur Herstellung von 1 : 1-Codimeren nach Anspruch 1, dadurch gekennzeichnet, daß man gasförmiges Butadien-1,3 mit Dicyclopentadien in Gegenwart einer kobaltorganischen Komplexverbindung bei 40—100° C reagieren läßt, wobei man den überwiegenden Anteil Butadien-1,3 höchstens im Maße seiner Reaktionsgeschwindigkeit zufügt.

3. Verfahren nach Anspruch 2, dadurch gekennzeichnet, daß die Reaktionstemperatur 50—70° C beträgt.

4. Verfahren nach Ansprüchen 2 und 3, dadurch gekennzeichnet, daß der Druck 1 bis 1,5 bar beträgt.

5. Verfahren nach Ansprüchen 2—4, dadurch gekennzeichnet, daß man das Butadien-1,3 in einer Menge von 0,05—0,5 Mol/h pro Mol vorgelegtes Dicyclopentadien zudosiert.

6. Verfahren nach Ansprüchen 2—5, dadurch gekennzeichnet, daß das Molverhältnis Butadien-1,3/Dicyclopentadien 0,1 bis 2 beträgt.

7. Verfahren nach Ansprüchen 2—6, dadurch gekennzeichnet, daß das Molverhältnis Butadien-1,3/Dicyclopentadien 1/3 bis 1 beträgt.

8. Verwendung der Codimeren nach Anspruch 1 als Reaktivverdünner für lufttrocknende Lacke.

**Claims**

1. 1 : 1-codimers of dicyclopentadiene and 1,3-butadiene having a molecular weight of 186 and the structure

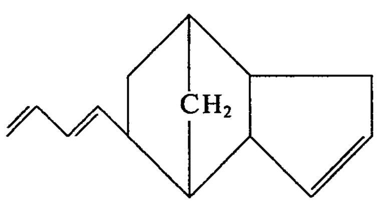

(I)

and/or

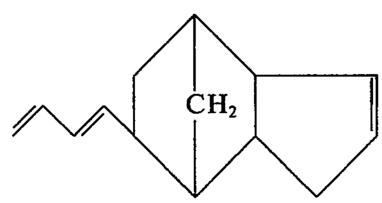

(II)

2. Process for producing 1 : 1-codimers according to Claim 1, characterised in that gaseous 1,3-butadiene is reacted with dicyclopentadiene in the presence of an organo-cobalt complex compound at 40—100° C, the major portion of the 1,3-butadiene being introduced at most at a rate commensurate with its reaction velocity.

3. Process according to Claim 2, characterised in that the reaction temperature is 50—70° C.

4. Process according to Claims 2 and 3, characterised in that the pressure is 1 to 1.5 bars.

5. Process according to Claims 2—4, characterised in that the 1,3-butadiene is added in a quantity of 0.05—0.5 mole/h per mole of dicyclopentadiene.

6. Process according to Claims 2—5, characterised in that the molar ratio of 1,3-butadiene to dicyclopentadiene is 0.1 : 1 to 2 : 1.

7. Process according to Claims 2—6, characterised in that the molar ratio of 1,3-butadiene to dicyclopentadiene is 1/3 : 1 to 1 : 1.

8. Use of the codimers according to Claim 1 as reactive thinners for air-drying lacquers.

**Revendications**

1. Codimères 1 : 1, de poids moléculaire 186, du dicyclopentadiène et du butadiène-1,3 de structure

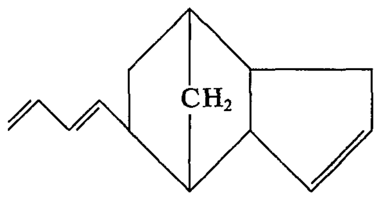

(I)

et/ou

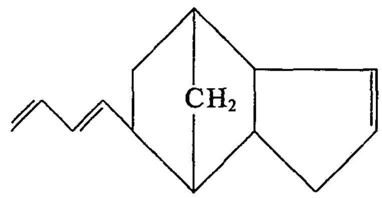

(II)

2. Procédé de préparation des codimères 1 : 1 selon la revendication 1, caractérisé en ce que l'on fait réagir du butadiène-1,3 gazeux avec du dicyclopentadiène en présence d'un complexe organique du cobalt à une température de 40 à 100°C, en ajoutant la plus grande partie du butadiène-1,3 au maximum en correspondance avec sa vitesse de réaction.

3. Procédé selon la revendication 2, caractérisé en ce que la température de réaction va de 50 à 70°C.

4. Procédé selon les revendications 2 et 3, caractérisé en ce que la pression va de 1 à 1,5 bar.

5. Procédé selon les revendications 2 à 4, caractérisé en ce que l'on ajoute le butadiène-1,3 en quantité de 0,05 à 0,5 moles/heure par mole de dicyclopentadiène introduit au préalable.

6. Procédé selon les revendications 2 à 5, caractérisé en ce que le rapport molaire butadiène-1,3/dicyclopentadiène va de 0,1 à 2.

7. Procédé selon les revendications 2 à 6, caractérisé en ce que le rapport molaire butadiène-1,3/dicyclopentadiène va de 1/3 à 1.

8. Utilisation des codimères selon la revendication 1, en tant que diluants réactifs pour des peintures et vernis séchant à l'air.